# EUROPEAN PATENT APPLICATION

(11) **EP 2 368 895 A1**
(43) Date of publication of application: **28.09.2011**
(21) Application number: 10305215.5
(22) Date of filing: 03.03.2010
(51) Int. Cl.: C07F 15/02, C07F 17/02, A61K 33/26, A61P 35/04

(54) **Ferrocenyl flavonoids**

(71) Applicant: Centre National de la Recherche Scientifique (CNRS), 75794 Paris Cedex 16 (FR)
(72) Inventor: Hillard, Elizabeth, 78370 Plaisir (FR); Chabot, Guy, 94220 Charenton Le Pont (FR); Monserrat, Jean-Philippe, 75001 Paris (FR); Jaouen, Gérard, 94230 Cachan (FR); Tiwari, Keshri Nath, 92340 Bourg La Reine (FR); De Montigny, Frédéric, 45200 Montargis (FR)
(74) Representative: Tarrère, Blandine

(57) **Abstract**

The present invention relates to new ferrocenyl flavonoids, processes for preparing them, pharmaceutical compositions containing them and their uses as pharmaceuticals.

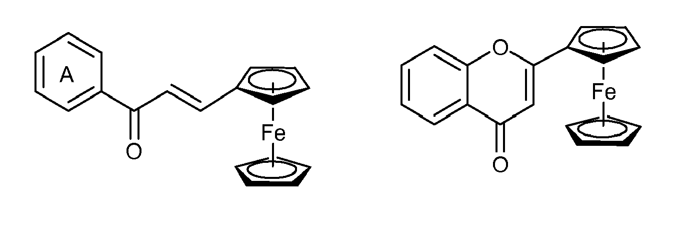

## Description

The present invention relates to new ferrocenyl compounds, processes for preparing them, pharmaceutical compositions containing them and their uses as pharmaceuticals.

The modification of the properties of biologically active molecules is currently an active field of research. Among others, modification by addition of ferrocene has given access to active ferrocenyl phenolic compounds.

Ferrocene, of formula Fe(C5H5)2, is a metallocene, a type of organometallic compound consisting of two cyclopentadienyl rings bound on opposite sides of a central metal atom, and in the case of ferrocene: an iron atom.

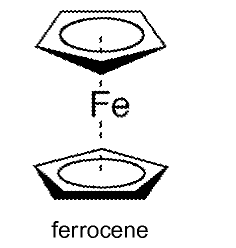

Flavonoids, such as flavanones and flavones, are ubiquitious plant-based polyphenols.

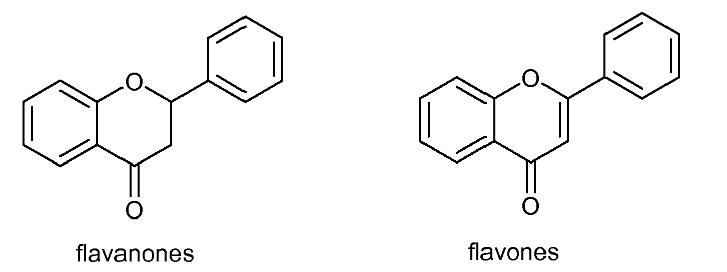

Their importance in health was first reported in 1936 by Rusznyak and Szent-Gyorgyi, and their numerous benefits have been reported in various conditions including cancer, cardio-vascular diseases, asthma, and viral infections. Several pathways for chemoprevention and anti-aging properties have been elucidated, particularly protective antioxidant properties. However, some flavonoids are also known to act as prooxidants because they can be metabolized to o-quinones and quinone methides which subsequently produce ROS (reactive oxygen species), which has been proposed as a way to stimulate apoptosis in cancer cells.

In plants, flavones and flavanones are biosynthesized from the precursor chalcones.

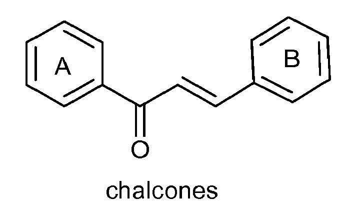

Such chalcones are usually experimentally obtained via base catalysed aldolic condensation, followed by cyclization in acidic conditions to form the flavanone or in the presence of I₂ to form the flavone (Cabrera et al. 2007).

Wu et al. (Bioorganic & Medicinal Chemistry Letters 12, 2002, 2299-2302) discloses some ferrocenyl chalcones having an antimalarial activity.

It is remarkable that only a few ferrocenyl chalcones have been studied. Additionally, although these few ferrocenyl chalcones have been widely studied for over 50 years, there are, to date, no reports of the corresponding ferrocenyl flavones or flavanones.

This lack of such compounds is probably due to the fact that such compounds can not be obtained by classical means. Indeed, the difficulty to form the corresponding flavones from the ferrocenyl chalcones arises from the electronic effects of the ferrocenyl unit, which deactivates the adjacent carbon atom to nucleophilic attack.

It has now surprisingly been found that the key step in the synthesis of ferrocenyl flavones involves a new 1,6-oxidative cyclization. Moreover, the toxicity of the resulting compounds has been tested and these compounds are among the most cytotoxic flavones known to date.

"Ferrocenyl flavones" and "ferrocenyl chalcones" usually refer to compounds having the following skeletons:

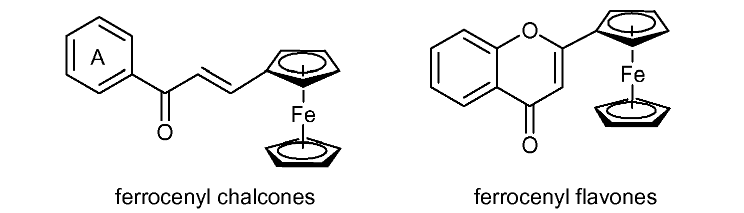

The invention provides new ferrocenyl flavones, in particular compounds of formula (I): 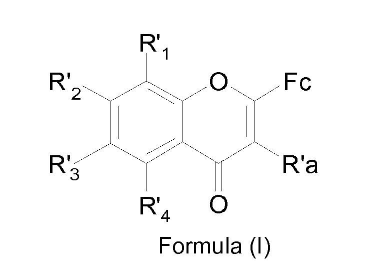 wherein
Fc is ferrocenyl,
R'a is hydrogen, a halogen, a C1-C6 alkyl, a C2-C6 alkenyl or a -CO-R'₅ group,
R'₁ is hydrogen, a halogen, hydroxy, nitro, a C1-C6 alkyl, a C2-C6 alkenyl or R'₁ together with R'₂ is a C6-C14 aryl,
R'₂ is hydrogen, a halogen, hydroxy, amino (-NH2), a C1-C6 alkyl, a C2-C6 alkenyl, -OR'₆, -NH-CO-R'₆, -O-CO-R'₆ or R'₂ together with R'₁ is a C6-C14 aryl,
R'₃ is hydrogen, a halogen, hydroxy, a C1-C6 alkyl, a C2-C6 alkenyl, -COOH, - OR'₇, -NH-CO-R'₇, -CO-R'₇ or R'₃ together with R'₄ is a C6-C14 aryl,
R'₄ is hydrogen, hydroxy, a C1-C6 alkyl, a C2-C6 alkenyl, -OR'₈ or R'₄ together with R'₃ is a C6-C14 aryl,
R'₅, R'₆, R'₇ and R'₈ are the same or different and are independently selected from a C1-C6 alkyl, a C2-C6 alkenyl or a C6-C14 aryl.

These ferrocenyl flavones show strong activity against cancer cells (see example 7).

The following paragraphs provide definitions and preferred embodiments are intended to apply uniformly throughout the specification and claims:
"C1-C6 alkyl", "C2-C6 alkenyl" refers respectively to a linear or branched alkyl comprising 1 to 6 carbon atom(s) or a linear or branched alkenyl comprising 2 to 6 carbon atom(s).

These terms are exemplified by groups such as methyl, ethyl, vinyl, n-propyl, allyl, isopropyl, iso-propenyl, n-butyl, isobutyl, tert-butyl, butenyl.

Preferably, alkyl and alkenyl comprise 1 to 4 carbon(s), more preferably, alkyl and alkenyl are linear and comprise 1 to 3 carbon(s).

"C6-C14 aryl" refers to an optionally substituted, unsaturated aromatic carbocyclic group of from 6 to 14 having a single ring or multiple condensed rings. Preferably, aryl include phenyl, naphtyl, bisphenyl, phenantrenyl and antracenyl, and more preferably, aryl is phenyl.

"Halogens" refers to fluoro, chloro, bromo and iodo. Preferred halogens are Cl, Br and F.

The invention also provides ferrocenyl chalcones, in particular compound of formula (II): 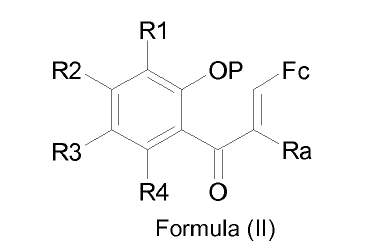 wherein
Fc is ferrocenyl,
P is hydrogen, an alcohol protecting group or -BF₂,
Ra is hydrogen, a halogen, a C1-C6 alkyl, a C2-C6 alkenyl or a -CO-R5 group,
R1 is hydrogen, a halogen, hydroxy, nitro, a C1-C6 alkyl, a C2-C6 alkenyl or R1 together with R2 is a C6-C14 aryl,
R2 is hydrogen, a halogen, hydroxy, amino (-NH₂), a C1-C6 alkyl, a C2-C6 alkenyl, -OR6, -NH-CO-R6, -O-CO-R6 or R2 together with R1 is a C6-C14 aryl,
R3 is hydrogen, a halogen, hydroxy, a C1-C6 alkyl, a C2-C6 alkenyl, -COOH, - OR7, -NH-CO-R7, -CO-R7 or R3 together with R4 is a C6-C14 aryl,
R4 is hydrogen, hydroxy, a C1-C6 alkyl, a C2-C6 alkenyl, -OR8 or R4 together with R3 is a C6-C14 aryl,
R5, R6, R7 and R8 are the same or different and are independently selected from a C1-C6 alkyl, a C2-C6 alkenyl or a C6-C14 aryl, with the proviso that the following compounds are excluded:
- 3-ferrocenyl-1-(2-hydroxyphenyl)-prop-2-en-1-one,
- 3-ferrocenyl-1-(2,4-dihydroxyphenyl)-prop-2-en-1-one,
- 3-ferrocenyl-1-(2,4-dimethoxyphenyl)-prop-2-en-1-one,
- 3-ferrocenyl-1-(2,3,4-trimethoxyphenyl)-prop-2-en-1-one.

Some of these ferrocenyl chalcones, 2-hydroxy ferrocenyl chalcones, are useful for the preparation of ferrocenyl flavones. Additionally, ferrocenyl chalcones also show a cytotoxic activity.

Preferred alcohol protecting groups are chosen among methoxy, ethoxy, propoxy, butoxy, tert-butoxy.

Preferably, when P is hydrogen or an alcohol protecting group:
- Ra is hydrogen, a halogen, a C1-C6 alkyl, a -CO-R5 group,
- R1 is hydrogen, a halogen, hydroxy, nitro, a C1-C6 alkyl or R1 together with R2 is a phenyl,
- R2 is hydrogen, a halogen, hydroxy, amino (-NH₂), a C1-C6 alkyl, -OR6, - NH-CO-R6, -O-CO-R6 or R2 together with R1 is a phenyl,
- R3 is hydrogen, a halogen, hydroxy, a C1-C6 alkyl, -COOH, -OR7, -NH-COR7, -CO-R7 or R3 together with R4 is a phenyl,
- R4 is hydrogen, hydroxy, a C1-C6 alkyl, -OR8 or R4 together with R3 is a phenyl,
- R5, R6, R7 and R8 are the same or different and are independently selected from a C1-C6 alkyl, a C2-C6 alkenyl or a phenyl,

In a specific embodiment, the ferrocenyl chalcones according to the invention are boron adducts when P is -BF₂.

Preferably, when P is -BF₂ in compounds of formula (II),
- Ra is hydrogen,
- R1 is hydrogen or a halogen,
- R2 is -OR6,
- R3 is hydrogen, a halogen or-OR7,
- R4 is -OR8,
- R6, R7 and R8 are the same or different and are independently a C1-C6 alkyl, preferably an unsubstituted C1-C6 alkyl, more preferably methyl.

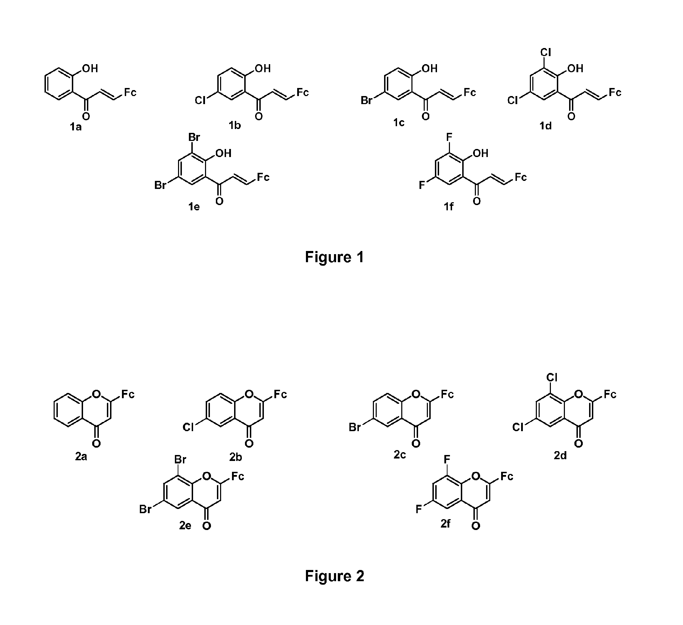

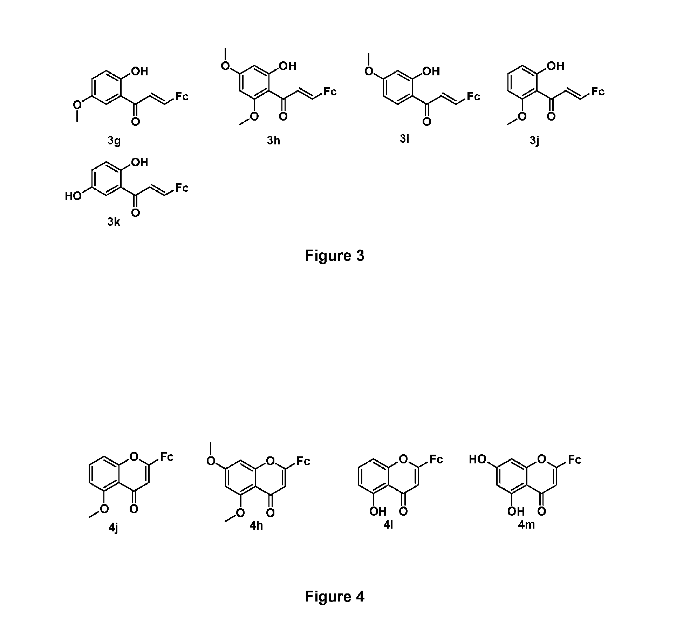

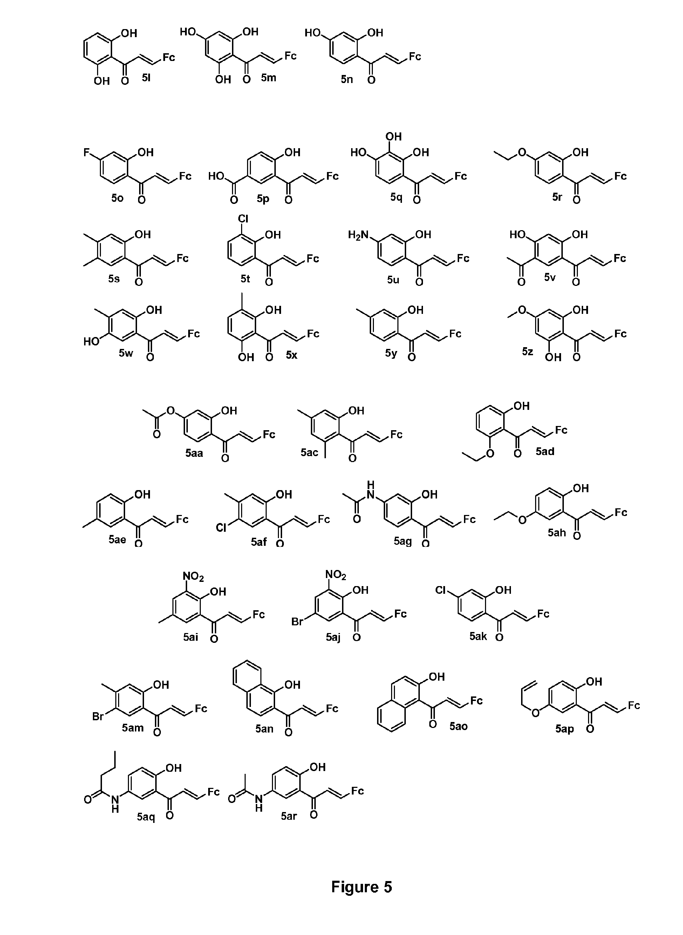

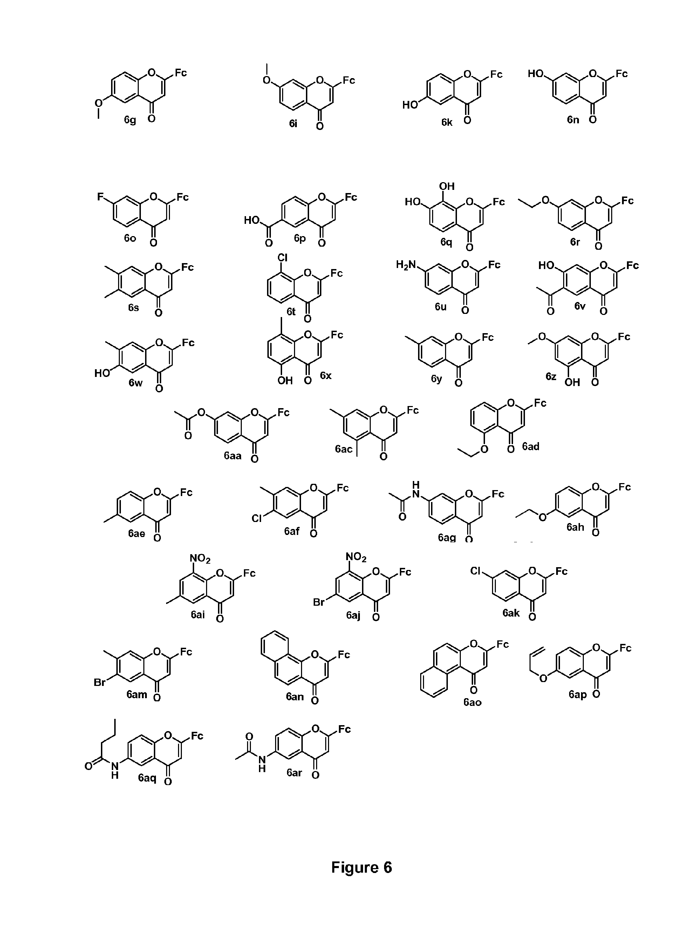

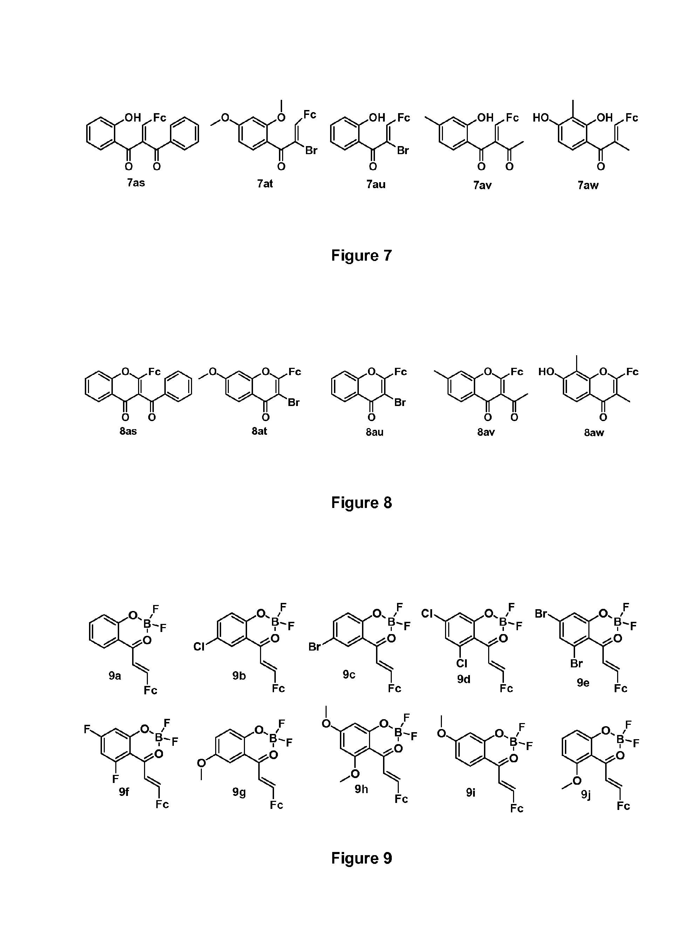

Preferred compounds according to the invention are (the corresponding developed formula being indicated in figures 1 to 9 in relation with the below-mentioned references):
1a : (E)-1-(2-hydroxyphenyl)-3-ferrocenylprop-2-en-1-one
1b : (E)-1-(5-chloro-2-hydroxyphenyl)-3-ferrocenylprop-2-en-1-one
1c : (E)-1-(5-bromo-2-hydroxyphenyl)-3-ferrocenylprop-2-en-1-one
1d : (E)-1-(3,5-dichloro-2-hydroxyphenyl)-3-ferrocenylprop-2-en-1-one
1e : (E)-1-(3,5-dibromo-2-hydroxyphenyl)-3-ferrocenylprop-2-en-1-one
1f : (E)-1-(3,5-difluoro-2-hydroxyphenyl)-3-ferrocenylprop-2-en-1-one
2a : 2-ferrocenyl-4H-chromen-4-one
2b : 6-chloro-2-ferrocenyl-4H-chromen-4-one
2c : 6-bromo-2-ferrocenyl-4H-chromen-4-one
2d : 6,8-dichloro-2-ferrocenyl-4H-chromen-4-one
2e : 6,8-dibromo-2-ferrocenyl-4H-chromen-4-one
2f : 6,8-difluoro-2-ferrocenyl-4H-chromen-4-one
3g : (E)-1-(2-hydroxy-5-methoxyphenyl)-3-ferrocenylprop-2-en-1-one
3h : (E)-1-(2-hydroxy-4,6-dimethoxyphenyl)-3-ferrocenylprop-2-en-1-one
3i : (E)-1-(2-hydroxy-4-methoxyphenyl)-3-ferrocenylprop-2-en-1-one
3j : (E)-1-(2-hydroxy-6-methoxyphenyl)-3-ferrocenylprop-2-en-1-one
3k : (E)-1-(2,6-dihydroxyphenyl)-3-ferrocenylprop-2-en-1-one
4j : 5-methoxy-2-ferrocenyl-4H-chromen-4-one
4h : 5,7-dimethoxy-2-ferrocenyl-4H-chromen-4-one
41 : 5-hyd roxy-2-ferrocenyl-4 H-chromen-4-one
4m : 5,7-dihydroxy-2-ferrocenyl-4H-chromen-4-one
5l : (E)-1-(2,6-dihydroxyphenyl)-3-ferrocenylprop-2-en-1-one
5m : (E)-3-ferrocenyl-1-(2,4,6-trihydroxyphenyl)prop-2-en-1-one
5n : (E)-1-(2,4-dihydroxyphenyl)-3-ferrocenylprop-2-en-1-one
5o : (E)-1-(4-fluoro-2-hydroxyphenyl)-3-ferrocenylprop-2-en-1-one
5p : (E)-4-hydroxy-3-(3-ferrocenylacryloyl)benzoic acid
5q : (E)-3-ferrocenyl-1-(2,3,4-trihydroxyphenyl)prop-2-en-1-one
5r : (E)-1-(4-ethoxy-2-hydroxyphenyl)-3-ferrocenylprop-2-en-1-one
5s : (E)-1-(2-hydroxy-4,5-dimethylphenyl)-3-ferrocenylprop-2-en-1-one
5t : (E)-1-(3-chloro-2-hydroxyphenyl)-3-ferrocenylprop-2-en-1-one
5u : (E)-1-(4-amino-2-hydroxyphenyl)-3-ferrocenylprop-2-en-1-one
5v: :(E)-1-(5-acetyl-2,4-dihydroxyphenyl)-3-ferrocenylprop-2-en-1-one
5w : (E)-1-(2,5-dihydroxy-4-methylphenyl)-3-ferrocenylprop-2-en-1-one
5x : (E)-1-(2,6-dihydroxy-3-methylphenyl)-3-ferrocenylprop-2-en-1-one
5y : (E)-1-(2-hydroxy-4-methylphenyl)-3-ferrocenylprop-2-en-1-one
5z : (E)-1-(2,6-dihydroxy-4-methoxyphenyl)-3-ferrocenylprop-2-en-1-one
5aa : (E)-3-hydroxy-4-(3-ferrocenylacryloyl)phenyl acetate
5ac : (E)-1-(2-hydroxy-4,6-dimethylphenyl)-3-ferrocenylprop-2-en-1-one
5ad : (E)-1-(2-ethoxy-6-hydroxyphenyl)-3-ferrocenylprop-2-en-1-one
5ae : (E)-1-(2-hydroxy-5-methylphenyl)-3-ferrocenylprop-2-en-1-one
5af : (E)-1-(5-chloro-2-hydroxy-4-methylphenyl)-3-ferrocenylprop-2-en-1-one
5ag : (E)-N-(3-hydroxy-4-(3-ferrocenylacryloyl)phenyl)acetamide
5ah : (E)-1-(5-ethoxy-2-hydroxyphenyl)-3-ferrocenylprop-2-en-1-one
5ai : (E)-1-(2-hydroxy-5-methyl-3-nitrophenyl)-3-ferrocenylprop-2-en-1-one
5aj : (E)-1-(5-bromo-2-hydroxy-3-nitrophenyl)-3-ferrocenylprop-2-en-1-one
5ak : (E)-1-(4-chloro-2-hydroxyphenyl)-3-ferrocenylprop-2-en-1-one
5am : (E)-1-(5-bromo-2-hydroxy-4-methylphenyl)-3-ferrocenylprop-2-en-1-one
5an : (E)-1-(1-hydroxynaphthalen-2-yl)-3-ferrocenylprop-2-en-1-one
5ao : (E)-1-(2-hydroxynaphthalen-1-yl)-3-ferrocenylprop-2-en-1-one
5ap : (E)-1-(5-(allyloxy)-2-hydroxyphenyl)-3-ferrocenyl)prop-2-en-1-one
5aq : (E)-N-(4-hydroxy-3-(3-ferrocenylacryloyl)phenyl)butyramide
5ar : (E)-N-(4-hydroxy-3-(3-ferrocenylacryloyl)phenyl)acetamide
6g : 6-methoxy-2-ferrocenyl-4H-chromen-4-one
6i : 7-methoxy-2-ferrocenyl-4H-chromen-4-one
6k : 6-hydroxy-2-ferrocenyl-4H-chromen-4-one
6n : 7-hydroxy-2-ferrocenyl-4H-chromen-4-one
6o 7-fluoro-2-ferrocenyl-4H-chromen-4-one
6p : 4-oxo-2-ferrocenyl-4H-chromene-6-carboxylic acid
6q : 7,8-dihydroxy-2-ferrocenyl-4H-chromen-4-one
6r : 7-ethoxy-2-ferrocenyl-4H-chromen-4-one
6s : 6,7-dimethyl-2-ferrocenyl-4H-chromen-4-one
6t : 8-chloro-2-ferrocenyl-4H-chromen-4-one
6u : 7-amino-2-ferrocenyl-4H-chromen-4-one
6v : 6-acetyl-7-hydroxy-2-ferrocenyl-4H-chromen-4-one
6w : 6-hydroxy-7-methyl-2-ferrocenyl-4H-chromen-4-one
6x : 5-hydroxy-8-methyl-2-ferrocenyl-4H-chromen-4-one
6y : 7-methyl-2-ferrocenyl-4H-chromen-4-one
6z : 5-hydroxy-7-methoxy-2-ferrocenyl-4H-chromen-4-one
6aa : 4-oxo-2-ferrocenyl-4H-chromen-7-yl acetate
6ac : 5,7-dimethyl-2-ferrocenyl-4H-chromen-4-one
6ad : 5-ethoxy-2-ferrocenyl-4H-chromen-4-one
6ae : 6-methyl-2-ferrocenyl-4H-chromen-4-one
6af : 6-chloro-7-methyl-2-ferrocenyl-4H-chromen-4-one
6ag : N-(4-oxo-2-ferrocenyl-4H-chromen-7-yl)acetamide
6ah : 6-ethoxy-2-ferrocenyl-4H-chromen-4-one
6ai : 6-methyl-8-nitro-2-ferrocenyl-4H-chromen-4-one
6aj : 6-bromo-8-nitro-2-ferrocenyl-4H-chromen-4-one
6ak : 7-chloro-2-ferrocenyl-4H-chromen-4-one
6am : 6-bromo-7-methyl-2-ferrocenyl-4H-chromen-4-one
6an : 2-ferrocenyl-4H-benzo[h]chromen-4-one
6ao : 3-ferrocenyl-1H-benzo[f]chromen-1-one
6ap : 6-(allyloxy)-2-ferrocenyl-4H-chromen-4-one
6aq : N-(4-oxo-2-ferrocenyl-4H-chromen-6-yl)butyramide
6ar : N-(4-oxo-2-ferrocenyl-4H-chromen-6-yl)acetamide
7as : (E)-1-(2-hydroxyphenyl)-2-(ferrocenylmethylene)-3-phenylpropane-1,3-dione
7at : (Z)-2-bromo-1-(2,4-dimethoxyphenyl)-3-ferrocenylprop-2-en-1-one
7au : (Z)-2-bromo-1-(2-hydroxyphenyl)-3-ferrocenylprop-2-en-1-one
7av : (E)-1-(2-hydroxy-4-methylphenyl)-2-(ferrocenylmethylene)butane-1,3-dione
7aw : (E)-1-(2,4-dihydroxy-3-methylphenyl)-2-methyl-3-ferrocenylprop-2-en-1-one
8as : 3-benzoyl-2-ferrocenyl-4H-chromen-4-one
8at : 3-bromo-7-methoxy-2-ferrocenyl-4H-chromen-4-one
8au : 3-bromo-2-ferrocenyl-4H-chromen-4-one
8av : 3-acetyl-7-methyl-2-ferrocenyl-4H-chromen-4-one
8aw : 7-hydroxy-3,8-dimethyl-2-ferrocenyl-4H-chromen-4-one
9a : (E)-1-(2-(difluoroboryloxy)phenyl)-3-ferrocenylprop-2-en-1-one
9b : (E)-1-(5-chloro-2-(difluoroboryloxy)phenyl)-3-ferrocenylprop-2-en-1-one
9c : (E)-1-(5-bromo-2-(difluoroboryloxy)phenyl)-3-ferrocenylprop-2-en-1-one
9d : (E)-1-(2,4-dichloro-6-(difluoroboryloxy)phenyl)-3-ferrocenylprop-2-en-1-one
9e : (E)-1-(2,4-dibromo-6-(difluoroboryloxy)phenyl)-3-ferrocenylprop-2-en-1-one
9f : (E)-1-(2,4-difluoro-6-(difluoroboryloxy)phenyl)-3-ferrocenylprop-2-en-1-one
9g : (E)-1-(2-(difluoroboryloxy)-5-methoxyphenyl)-3-ferrocenylprop-2-en-1-one
9h : (E)-1-(2-(difluoroboryloxy)-4,6-dimethoxyphenyl)-3-ferrocenylprop-2-en-1-one
9i : (E)-1-(2-(difluoroboryloxy)-4-methoxyphenyl)-3-ferrocenylprop-2-en-1-one
9j : (E)-1-(2-(difluoroboryloxy)-6-methoxyphenyl)-3-ferrocenylprop-2-en-1-one

As mentioned previously, preparation of ferrocenyl flavones starts from 2-hydroxy ferrocenyl chalcones and involves a new 1,6-oxidative cyclization.

Thus a method of preparation of ferrocenyl flavones is disclosed, by oxidation of a compound of formula (III): 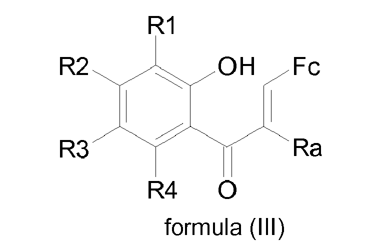 wherein
Fc is ferrocenyl,
Ra is hydrogen, a halogen, a C1-C6 alkyl, a C2-C6 alkenyl or a -CO-R5 group,
R1 is hydrogen, a halogen, hydroxy, nitro, a C1-C6 alkyl, a C2-C6 alkenyl or R1 together with R2 is a C6-C14 aryl,
R2 is hydrogen, a halogen, hydroxy, amino (-NH2), a C1-C6 alkyl, a C2-C6 alkenyl, -OR6, -NH-CO-R6, -O-CO-R6 or R2 together with R1 is a C6-C14 aryl,
R3 is hydrogen, a halogen, hydroxy, a C1-C6 alkyl, a C2-C6 alkenyl, -COOH, - OR7, -NH-CO-R7, -CO-R7 or R3 together with R4 is a C6-C14 aryl,
R4 is hydrogen, hydroxy, a C1-C6 alkyl, a C2-C6 alkenyl, -OR8 or R4 together with R3 is a C6-C14 aryl,
R5, R6, R7 and R8 are the same or different and are independently selected from a C1-C6 alkyl, a C2-C6 alkenyl or a C6-C14 aryl,
in the presence of strong base and an oxidizing agent.

This method gives acess to a new class of cytotoxic compounds, ferrocenyl flavones. The reaction is very simple, involves the use of relatively inexpensive reagents, and can be carried out in air, at room temperature, in one pot. The reaction yield is usually quantitative, with more than 90% conversion of the precursor 2-hydroxy ferrocenyl chalcone.

Advantageously, the strong base is chosen among sodium hydride, potassium t-butoxide, calcium hydride, sodium hydroxide and the oxidizing agent is chosen among silver salts.

Preferably, the strong base is sodium hydride and the oxidizing agent is silver triflate, silver tetrafluoroborate and silver hexafluorophosphate.

In a preferred embodiment, the oxidation is conducted in an organic solvent chosen among THF or ethanol.

The invention further relates to a method of preparation of a ferrocenyl chalcone boron adducts, by reacting a compound of formula (III): 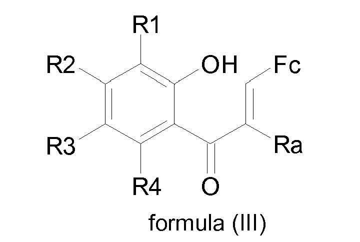 wherein
Fc is ferrocenyl,
Ra is hydrogen, a halogen, a C1-C6 alkyl, a C2-C6 alkenyl or a -CO-R5 group,
R1 is hydrogen, a halogen, hydroxy, nitro, a C1-C6 alkyl, a C2-C6 alkenyl or R1 together with R2 is a C6-C14 aryl,
R2 is hydrogen, a halogen, hydroxy, amino (-NH2), a C1-C6 alkyl, a C2-C6 alkenyl, -OR6, -NH-CO-R6, -O-CO-R6 or R2 together with R1 is a C6-C14 aryl,
R3 is hydrogen, a halogen, hydroxy, a C1-C6 alkyl, a C2-C6 alkenyl, -COOH, - OR7, -NH-CO-R7, -CO-R7 or R3 together with R4 is a C6-C14 aryl,
R4 is hydrogen, hydroxy, a C1-C6 alkyl, a C2-C6 alkenyl, -OR8 or R4 together with R3 is a C6-C14 aryl,
R5, R6, R7 and R8 are the same or different and are independently selected from a C1-C6 alkyl, a C2-C6 alkenyl or a C6-C14 aryl,
with a strong base, and adding to the reaction mixture (boron trifluoride.solvent) and a strong acid. Advantageously, the (boron trifluoride.solvent) is chosen among (boron trifluoride.diethyl etherate) or (boron trifluoride.dimethylsulfide).

Advantageously, the strong base is chosen among metal hydrides such as sodium or calcium hydride and the strong acid is chosen among strong mineral acids such as hydrochloric, hydrobromic or sulfuric acid. Preferably, the strong base is sodium hydride and the strong acid, hydrochloric acid.

The compounds of the present invention, and the pharmaceutically acceptable salts thereof, are for use as a medicament, in particular for the treatment of cancer, Cancer that can be treated by the compounds according to the invention are more particularly melanoma, breast, prostate, lung, bladder, pancreas, kidney and brain cancers.

The "pharmaceutically acceptable salt" according to the invention include therapeutically active, non-toxic acid or base salts forms which the compounds according to the invention are able to form.

In another embodiment, the boron adducts according to the invention (i.e., compounds of formula II wherein P is -BF₂) are also for use as a medicament for the treatment of HIV.

A further aspect of the invention relates to a pharmaceutical composition comprising an effective amount of a compound according to the invention in combination with a pharmaceutically acceptable diluent or carrier.

Other characteristics and advantages of the invention will be made clear in the following examples, with reference to the drawings, in which, respectively:
- figures 1 to 9 show the developed formula of preferred compounds according to the invention,
- figure 10 is a chart that illustrates the ferrocenyl flavones cytotoxicity.

### Example 1: Identification of the new 1,6-oxidative cyclization of ferrocenyl chalcones to obtain corresponding flavones

Ferrocenyl chalcones 1a-f, possessing a hydroxyl group in the 2' position, were synthesized by the standard method, by combining an equimolar proportion of ferrocene carboxyaldehyde and the appropriate 2-hydroxyacetophenone in the presence of three equivalents NaOH in EtOH and stirring at room temperature overnight. The deep violet products can be separated from unreacted starting material via a silica gel column using petroleum ether and dichloromethane (4:1), with yields of 60-70% after purification.

The transformation from chalcone to flavanone involves a 1,4 intramolecular nucleophilic addition, and simply refluxing the starting chalcone in acetic acid is the standard method. However, 1 a was completely unreactive under these conditions, and other methods, such as the use of basic conditions or Lewis acids were likewise unsuccessful. This is likely why, although many groups have studied ferrocenyl chalcones, nobody yet has been able to synthesize the ferrocenyl flavones or flavanones.

The unreactivity of 1a is probably due to the reduced electrophilicity of the 4 position, due to the electron donating properties of the ferrocene group. Thus, the oxidation of the ferrocene group to a ferricenium group was thought to be able make the 4 position susceptible to nucleophilic attack. Consequently, 1a was dissolved in THF and 2.5 eq NaH was added. The violet solution quickly became light red in color as the phenolate formed. One equivalent of the oxidizing agent silver triflate was then added, and a black precipitate formed, indicating the formation of elemental silver. After 5 minutes of stirring, TLC indicated the formation of a new, blue product, which was more polar than 1a. Unexpectedly, the new product was identified as the ferrocenyl flavone (scheme 1). The 1H NMR spectra in deuterated chloroform indicated the disappearance of the vinylic signal of 1a, (2 doublets at 7.93 and 7.88 ppm with a coupling constant of 15.1 Hz) and the appearance of a singlet integrating for 1 H at 6.89 ppm. Its mass spectrum (APCI) exhibited a molecular ion at m/z = 331.07 [M+H] + corresponding to the ferrocene flavone. The conversion of the chalcones to the corresponding flavones was > 95% by 1H HMR.

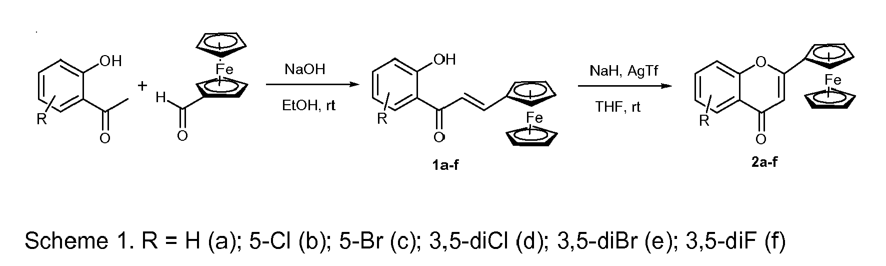

The conversion from chalcone to flavone can therefore be considered as an oxidative cyclization involving the loss of two electrons and two protons, instead of a simple intramolecular nucleophilic attack. Indeed, when the oxidant was increased to 2.2 eq, the reaction was quantitative. This is the first time ferrocene was used as an intramolecular catalyst for such a reaction. The transformation from the chalcones to the flavone is fast (10 minutes), giving >90% conversion, can be performed at room temperature in standard air conditions. The most expensive reagent is silver triflate, which costs approximately 50 euros/10 g.

### Example 2: Synthesis of ferrocenyl chalcones 1 a-f

General preparation of ferrocenyl chalcones: Ferrocene carboxaldehyde (1 eq) and the appropriate 2-hydroxyacetophenone (1 eq) were dissolved in absolute ethanol (40 mL) in a 100 mL two necked round bottom flask. After stirring the mixture 10 to 15 min. at room temperature, sodium hydroxide (3 eq) was added, and the solution was stirred overnight. The mixture was poured into water (100 mL) and hydrochloric acid (12 M, 15 mL), extracted with dichloromethane (3 x 50 mL), and washed with water. The organic phase was dried over magnesium sulfate, filtered, and the solvent removed by evaporation. The product was purified by silica gel chromatography, using a mixture of petroleum ether/dichloromethane 4:1 as an eluent, and again using HPLC in acetonitrile/water (90:10). After HPLC purification, the acetonitrile was removed under reduced pressure and the aqueous phase extracted with dichloromethane.

**(E)-1-(2-hydroxyphenyl)-3-ferrocenylprop-2-en-1-one, 1a.** Violet solid. NMR 1H (300 MHz, CDCl3): δ 7.90 (d, J=15.1 Hz, 1H, Hvinyl), 7.86 (d, J=7.7 Hz, 1H, Har), 7.48 (t, J=7.8 Hz, 1H, Har), 7.25 (d, J=15.1 Hz, 1H, Hvinyl), 7.02 (d, J=7.8 Hz, 1H, Har), 6.93 (t, J=7.7 Hz, 1H, 1 Har), 4.64 (s, 2H, C5H4), 4.55 (s, 2H, C5H4), 4.20 (s, 5H, C5H5). NMR 13C (75 MHz, CDCl3): δ 192.7 (Cketone), 162.6 (Car), 147.9 (Cvinyl), 135.9 (Car), 129.3 (Car), 120.0 (Cvinyl), 118.7 (Car), 118.6 (Car), 116.7 (Car), 78.9 (C5H4, Cquad), 71.8 (C5H4), 69.9 (C5H5), 69.3 (C5H4). MS (APCI) m/z 332.07 [M+H]+, Anal. Calcd for C19H16O2Fe.0.35 dichloromethane: C 64.22, H 4.65. Found: C 64.43, H 4.89.

**(E)-1-(5-chloro-2-hydroxyphenyl)-3-ferrocenylprop-2-en-1-one, 1b.** Violet solid. NMR 1H (300 MHz, CDCl3): δ 7.94 (d, J=15 Hz, 1H, Hvinyl), 7.80 (d, J=2.4 Hz, 1H, Har), 7.42(dd, 3J=9 Hz, 4J=2.4 Hz, 1H, Har), 7.14 (d, J=15 Hz, 1H, Hvinyl), 6.96 (d, J=9 Hz, 1H, Har), 4.66 (s, 2H, C5H4), 4.51 (s, 2H, C5H4), 4.22 (s, 5H, C5H5). NMR 13C (75 MHz, CDCl3): δ 191.7 (Cketone), 162.1 (Car), 149.4 (Cvinyl), 135.6 (Car), 128.5 (Car), 123.3 (Cvinyl), 120.7 (Car), 120.2 (Car), 115.9 (Car), 78.6 (C5H4, Cquad), 72.2 (C5H4), 70.0 (C5H5), 69.5 (C5H4). MS (APCI) m/z 366.03 [M+H]+, Anal. Calcd for C19H15O2FeCl.0.06 dichloromethane: C 61.59, H 4.1. Found: C 61.75, H 4.02.

**(E)-1-(5-bromo-2-hydroxyphenyl)-3-ferrocenylprop-2-en-1-one, 1c.** Violet solid. NMR 1H (300 MHz, CDCl3): δ 7.94 (d, J=14.4 Hz, 1H, Hvinyl), 7.92 (m, 1H, Har), 7.55 (d, J=8.1 Hz, 1H, Har), 7.13 (d, J=14.5 Hz, 1H, Hvinyl), 6.92 (d, J=8.1 Hz, 1H, Har), 4.66 (s, 2H, C5H4), 4.58 (s, 2H, C5H4), 4.22 (s, 5H, C5H5). NMR 13C (75 MHz, CDCl3): δ 191.6 (Cketone), 162.5 (Car), 149.4 (Cvinyl), 138.4 (Car), 131.6 (Car), 121.3 (Cvinyl), 120.6 (Car), 115.9 (Car), 110.3 (Car), 78.6 (C5H4, Cquad), 72.3 (C5H4), 70.1 (C5H5), 69.5 (C5H4). MS (APCI) m/z 410.05 [M+H]+, Anal. Calcd for C19H15O2FeBr.0.05 dichloromethane: C 55.09, H 3.66. Found: C 55.04, H 3.73.

**(E)-1-(3,5-dichloro-2-hydroxyphenyl)-3-ferrocenylprop-2-en-1-one,** 1d. Violet solid. NMR 1H (300 MHz, CDCl3): δ 8.00 (d, J=15 Hz, 1H, Hvinyl), 7.74 (s, 1H, Har), 7.57 (s, 1H, Har), 7.01 (d, J=15 Hz, 1H, Hvinyl), 4.67 (s, 2H, C5H4), 4.62 (s, 2H, C5H4), 4.23 (s, 5H, C5H5). NMR 13C (75 MHz, CDCl3): δ 190.2 (Cketone), 157.0 (Car), 149.8 (Cvinyl), 134.1 (Car), 126.1 (Car), 122.9 (Car), 122.0, (Car) 120.2 (Car), 114.2 (Cvinyl), 77.3 (C5H4, Cquad), 71.6 (C5H4), 69.1 (C5H5), 68.7 (C5H4). MS (APCI) m/z 400.08 [M+H]+, Anal. Calcd for C19H14O2FeCl2·0.125 dichloromethane: C 55.80, H 3.49. Found: C 55.72, H 3.49.

**(E)-1-(3,5-dibromo-2-hydroxyphenyl)-3-ferrocenylprop-2-en-1-one, 1e.** Violet solid. NMR 1H (300 MHz, CDCl3): δ 7.99 (d, J=14.7 Hz, 1H, Hvinyl), 7.91 (d, 4J=1.8 Hz, 1H, Har), 7.86 (d, 4J=1.8 Hz, 1H, Har), 7.10 (d, J=14.7 Hz, 1H, Hvinyl), 4.67 (s, 2H, C5H4), 4.62 (s, 2H, C5H4), 4.23 (s, 5H, C5H5). NMR 13C (75 MHz, CDCl3): δ 191.0 (Cketone), 159.3 (Car) 150.8 (Cvinyl), 140.7 (Car), 130.8 (Car), 121.7 (Car), 115.1 (Cvinyl), 113.3(Car), 110.1 (Car), 78.37 (C5H4, Cquad) 72.6 (C5H4), 70.1 (C5H5), 69.7 (C5H4). MS (APCI) m/z 488.01 [M+H]+, Anal. Calcd for C19H14O2FeBr2.0.25 dichloromethane: C 45.23, H 2.86. Found: C 45.24, H 2.70.

**(E)-1-(3,5-difluoro-2-hydroxyphenyl)-3-ferrocenylprop-2-en-1-one, 1f.** Violet solid. NMR 1H (300 MHz, CDCl3): δ 7.98 (d, J=15 Hz, 1H, Hvinyl), 7.34 (s, 1H, Har), 7.06 (m, 2H, Hvinyl, Har) , 4.66 (s, 2H, C5H4), 4.61 (s, 2H, C5H4), 4.22 (s, 5H, C5H5). NMR 13C (100 MHz, CDCl3): δ 191.6 (Cketone), 153.3 (dd, 1J=238.6 Hz, 3J=9 Hz, Car), ∼151 (Car overlap) 150.5 (Cvinyl), 148.8 (d, 2J=11.9 Hz, Car) 120.9 (Car), 115.7 (Cvinyl), 110.5 (dd, 2J=26 Hz, 2J=21 Hz, Car), 109.6 (d, 2J=23 Hz, Car), 78.4 (C5H4, Cquad), 72.6 (C5H4), 70.2 (C5H5), 69.7 (C5H4). MS (APCI) m/z 368.07 [M+H]+, Anal. Calcd for C19H14O2FeF2.0.2 dichloromethane: C 59.88, H 3.77. Found: C 60.25, H 4.04.

### Example 3: Synthesis of ferrocenyl flavones 2 a-f

General preparation of ferrocenyl flavones: Ferrocenyl chalcone (1 eq) was dissolved in tetrahydrofuran (15 mL) in a 50 mL two-necked round bottom flask. Sodium hydride (3 eq) was added and the solution went from deep violet to a light red. After stirring for 5 min, AgOTf (2.5 eq) was added, and the mixture was stirred for another 5 min, before being poured into a water (100 mL) and HCl 12 M (15 mL). The mixture was extracted with dichloromethane (3 x 50 mL), and washed with water. The organic phase was dried over magnesium sulfate, filtered and evaporated. The product was purified using a silica gel column, using a mixture of petroleum ether/dichloromethane 1/1, and again by HPLC using acetonitrile/water, and the 1H NMR spectra showed the presence of residual water. Yields were calculated after purification on the silica gel column.

**2-ferrocenyl-chromen-4-one,** 2a. Violet solid. Yield: 80%. NMR 1H (300 MHz, CDCl3): δ 7.80 (d, J=7.5 Hz, 1H, Har), 7.64 (t, J=7.2 Hz, 1H, Har), 7.29 (d, J=7.2 Hz, 1H, Har), 7.19 (t, J=7.5 Hz 1H, Har), 6.89 (s, 1H, Hvinyl), 4.92 (s, 2H, C5H4), 4.60 (s, 2H, C5H4), 4.22 (s, 5H, C5H5). NMR 13C (75 MHz, CDCl3): δ 182.9 (Cketone), 165.4 (Car), 146.0 (Cvinyl), 136.1 (Car), 124.5 (Car), 123.1 (Cvinyl), 122.6 (Car), 116.4 (Car), 112.9 (Car), 75.1 (C5H4, Cquad), 71.8 (C5H4), 71.5 (C5H4), 70.0 (C5H5). MS (APCI) m/z 331.07 [M+H]+. Anal. Calcd for C19H14O2Fe.0.5 water: C 67.28, H 4.46. Found: C 67.60, H 4.66.

**6-chloro-2-ferrocenyl-chromen-4-one,** 2b. Violet solid. Yield: 78% NMR 1H (300 MHz, CDCl3): δ 7.77 (d, J=2.1 Hz, 1H, Har), 7.57 (dd, 4J=2.1 Hz, 3J=8.7 Hz, 1H, Har), 7.24 (m, 1H, Har), 6.92 (s, 1H, Hvinyl), 4.91 (s, 2H, C5H4), 4.63 (s, 2H, C5H4), 4.24 (s, 5H, C5H5). NMR 13C (75 MHz, CDCl3): δ 181.4 (Cketone), 163.5 (Car), 146.1 (Cvinyl), 135.8 (Car), 128.8 (Car), 124.0 (Cvinyl), 123.8 (Car), 117.9 (Car), 114.2 (Car), 74.7 (C5H4, Cquad), 72.2 (C5H4), 71.6 (C5H4), 70.0 (C5H5). MS (APCI) m/z 364.02 [M+H]+. Anal. Calcd for C19H13O2FeCl.0.25 water: C 61.66, H 3.69. Found: C 61.65, H 3.67.

**6-bromo-2-ferrocenyl-chromen-4-one,** 2c. Violet solid. Yield: 64%. NMR 1H (300 MHz, CDCl3): δ 7.93 (d, J=2.1Hz, 1H, Har), 7.72 (dd, 4J=2.1Hz, 3J=8.7 Hz, 1H, Har), 7.20 (d, J=8.7 Hz, 1H, Har), 6.94 (s, 1H, Hvinyl), 4.86 (m, 2H, C5H4), 4.60 (m, 2H, C5H4), 4.19 (s, 5H, C5H5). NMR 13C (75 MHz, CDCl3): δ 181.2 (Cketone), 163.9 (Car), 145.9 (Cvinyl), 138.5 (Car), 127.1 (Car), 124.4 (Cvinyl), 118.0 (Car), 115.9 (Car), 114.7 (Car), 74.7 (C5H4, Cquad), 72.2 (C5H4), 71.7 (C5H4), 70.1 (C5H5). MS (APCI) m/z 408.08 [M+H]+. Anal. Calcd for C19H13O2FeBr.0.165 water: C 55.39, H 3.26. Found: C 55.77, H 3.65.

**6,8-dichloro-2-ferrocenyl-chromen-4-one,** 2d. Blue solid. Yield: 62%. NMR 1H (300 MHz, CDCl3): δ 7.69 (d, J=2.1 Hz, 1H, Har), 7.64 (d, J=2.1 Hz, 1H, Har), 7.06 (s, 1H, Hvinyl), 4.93 (s, 2H, C5H4), 4.66 (s, 2H, C5H4), 4.22 (s, 5H, C5H5). NMR 13C (75 MHz, CDCl3): δ 179.3 (Cketone), 158.2 (Car), 144.8 (Cvinyl), 134.0 (Car), 127.9 (Car), 124.1 (Car), 121.4 (Cvinyl), 118.9 (Car), 118.4 (Car), 73.3 (C5H4, Cquad), 71.7 (C5H4), 71.0 (C5H4), 69.2 (C5H5). MS (APCI) m/z 398.00 [M+H]+. Anal. Calcd for C19H12O2FeCl2.0.5 water: C 55.93, H 3.21. Found: C 56.05, H 3.31.

**6,8-dibromo-2-ferrocenyl-chromen-4-one,** 2e. Blue solid. Yield: 80%. 1H NMR (300 MHz, CDCl3): δ 7.92 (d, J=1.5 Hz, 1H, Har), 7.86 (d, J=1.5 Hz, 1H, Har), 7.03 (s, 1H, Hvinyl), 4.93 (s, 2H, C5H4), 4.67 (s, 2H, C5H4), 4.22 (s, 5H, C5H5). NMR 13C (75 MHz, CDCl3): δ 179.3 (Cketone), 159.9 (Car), 144.4 (Cvinyl), 139.2 (Car), 129.8 (Car), 125.0 (Cvinyl), 124.5 (Car), 119.0 (Car), 115.0 (Car), 73.3 (C5H4, Cquad), 71.7 (C5H4), 71.0 (C5H4), 69.2 (C5H5). MS (APCI) m/z 485.99 [M+H]+. Anal. Calcd for C19H12O2FeBr2.0.25 water: C 46.34, H 2.56. Found: C 46.47, H 2.60.

**6,8-difluoro-2-ferrocenyl-chromen-4-one,** 2f. Violet-blue solid. Yield: 86%. NMR 1H (300 MHz, CDCl3): δ 7.31-7.29 (m, 1H, Har), 7.22 -7.15 (m, 1H, Har), 7.02 (s, 1H, Hvinyl), 4.91 (s, 2H, C5H4), 4.65 (s, 2H, C5H4), 4.22 (s, 5H, C5H5). NMR 13C (100 MHz, CDCl3): δ 180.7 (Cketone), 157.9 (dd, 1J=246.5 Hz, 3J=7.3 Hz, Car), 148.8 (d, 2J=11.3 Hz, Car), 148.1 (dd, 1J=254.7 Hz, 3J=11.2 Hz, Car), 145.9 (Cvinyl), 125.6 (d, 3J=7.7 Hz, Car), 119.8 (Cvinyl), 110.9 (dd, 2J=19.7 Hz, 2J=24.3 Hz, Car), 105.5 (dd, 2J=23.8 Hz, 4J=4.1 Hz, Car), 74.3 (C5H4, Cquad), 72.6 (C5H4), 71.9 (C5H4), 70.2 (C5H5). MS (APCI) m/z 366.04 [M+H]+. Anal. Calcd for C19H12O2FeF2.0.5 water: C 60.83, H 3.49. Found: C 61.04, H 3.22.

### Example 4: Synthesis of flavones 4i and 4h

Synthesis:
A 100 mL round-bottom flask was equipped with a magnetic stirring bar and a rubber septum. The flask was charged with 0.200 g of the corresponding chalcone in 50 mL of ethanol. 0.300 g of potassium tertbutoxide (3 eq) is added and 0.500 g of silver triflate was added in three times every 10 min. The reaction mixture was stirred 2 h. 30 mL of an aqueous solution of HCl (12M) was added to the flask and the reaction mixture was transferred to a 250 mL separatory funnel. The organic phase was separated. The aqueous layer was extracted with two 100 mL portions of dichloromethane and the combined organic extracts were dried over anhydrous magnesium sulphate, filtered, and concentrated under reduced pressure. The residue was purified by silica gel column chromatography.
Results:
**4h :** MS Cl(NH4+) [M+H+]=391
**4j :** Violet solid. RMN 1H (300 MHz, CDCl3): δ 7.48 (t, J= 15.3 Hz, 1H, Har), 6.75 (d, J= 15.3 Hz, 1H, Har), 6.72 (s, 1H, Hvinyl), 6.53 (d, J=15.3 Hz, 1H, Har), 4.83 (s, 2H, C5 H4), 4.51 (s, 2H, C5 H4), 4.20 (s, 5H, C5H4), 3.93 (s, 3H, OCH3). MS CI(NH4+) [M+H+]=361

### Example 5: Synthesis of flavones 41 and 4m

Synthesis:
A 100 mL round-bottom flask was equipped with a large magnetic stirring bar and a rubber septum. The flask was charged with 0.200 g of the corresponding methoxyflavone in 50 mL of distilled dichloromethane. 0.7 mL of BBr3 (3 equivalents) was added very slowly to the mixture under argon. After 2h, an aqueous solution of sodium hydroxide was added to the reaction mixture until a basic pH was reached. The residue was transferred to a 250 mL seperatory funnel and the aqueous layer was washed three times with 100 mL of dichloromethane. The combined organic extracts were dried over anhydrous magnesium sulfate, filtered, and concentrated under reduced pressure. The residue was purified by silica gel chromatography.
Results:
**4l** : Violet solid. RMN 1H (300 MHz, CDCl3): δ 7.9 (t, J= 15 Hz, 1H, Har), 7.55 (s, 1H, Hvinyl), 6.9 (d, J=15Hz, 1H, Har), 6.85 (d, J=15 Hz, 1H, Har), 6.6 (s, 2H, C5 H4), 4.9 (s, 2H, C5 H4), 4.6 (s, 2H, C5H4), 4.2 (s, 5H, C5H4). MS Cl(NH4+) [M+H+]=347
**4m :** Violet solid. RMN 1H (300 MHz, CDCl3): δ 7.67 (s, 1H, Hvinyl), 6.29 (d, J= 15.3 Hz, 1H, Har ), 6.05 (d, J= 15.3 Hz, 1H, Har), 4.73 (s, 2H, C5H5), 4.41 (s, 2H, C5H4), 4.09 (s, 5H, C5H4), 3.84 (s, 3H, OCH3) 3.83 (s, 3H, OCH3).

### Example 6: Synthesis of boron adducts

Synthesis:
To a solution of chalcone (approximately 300 mg scale) in 50 mL of distilled CH2Cl2 was added 3 equivalents of NaH under argon. The reaction mixture was stirred for 10 minutes. Then 3 equivalents of BF₃-OEt₂ was added dropwise to the reaction mixture, which immediately became green. To the reaction mixture was added a solution of hydrochloric acid 37% in water, and the mixture was separated. The aqueous phase was washed 3 times with 100 mL CH₂Cl₂. The organic phase was dried over MgSO₄, and distilled off using a rotary evaporator at 650 mbar. The obtained green crystals were dried in vacuum and purified by column chromatography, with silica gel as a stationary phase and a 3:1 mixture of dichloromethane and petroleum spirit as an eluent. The crystals were purified by recrystallization: crystals were dissolved in a few drops of dichloromethane, and a large excess of petroleum spirit was added on the mixture. The mixture was then placed at 4°C during 10 minutes, filtered by means of a Büchner funnel and rinsed with petroleum spirit.
Results:
**9a** : Green solid. TLC (dichloromethane/petroleum spirit 3:1): Rf=0,237. MS (APCI) m/z 457.96 [M]+°. Yield=80%.
**9b** : Green solid. TLC (dichloromethane/petroleum spirit 3:1): Rf=0,312. NMR 1H (300 MHz, CDCl3): 4.377 (s, 5H, H1 ferrocene), 4.890 (s, 2H, H2 or H3 ferrocene), 5.079 (s, 2H, H2 or H3 ferrocene), 6.958 (d, 1H, H6 aromatic, J=8,7 MHz), 7.088 (d, 1H, H4 or H5 vinyl, J=14,1 MHz), 7.629 (d, 1H, H7 aromatic, J=8,3 MHz), 7.786 (s, 1H, H8 aromatic), 8.693 (d, 1H, H4 or H5 vinyl, J=14,1 MHz). NMR 13C (75 MHz, CDCl3): 72.3 (C1 ferrocene), 79.3 (Cquat ferrocene), 110.6 (C aromatic), 123.4 (C aromatic), 125.4 (Cquat aromatic), 127.4 (C4 or C5 vinyl), 140.4 (C aromatic), 161.5 (C aromatic), 162.2 (Cquat aromatic), 182.2 (Cquat ketone). MS (APCI) m/z 414.04 [M]+°. Yield=85%.
**9c** : Green solid. TLC (dichloromethane/petroleum spirit 3:1): Rf=0,231. NMR 1H (300 MHz, CDCl3): 4.28 (s, 5H, H1 ferrocene), 4.79 (s, 2H, H2 or H3 ferrocene), 4.98 (s, 2H, H2 or H3 ferrocene), 6.84 (d, 1H, H6 aromatic, J=8.8 MHz), 6.89 (d, 2H, H4 or H5 vinyl, J=13.9 MHz), 7.46 (d, 1H, H7 aromatic, J=8.5 MHz), 7.84 (m, 1H, H8 aromatic), 8.61 (d, 1H, H4 or H5 vinyl, J=14.0 MHz). NMR 13C (75 MHz, CDCl3): 71.8 (C1 ferrocene), 78.8 (Cquat ferrocene), 110.3 (C aromatic), 112.1 (Cquat aromatic), 120.6 (C aromatic), 123.3 (C4 or C5 vinyl), 130.2, 131.7 (C aromatic), 138.4 (C aromatic), 161.2 (C4 or C5 vinyl), 162.5 (Cquat aromatic). MS (APCI) m/z 457.96 [M]+°. Yield=98%.
**9d** : Green solid. TLC (dichloromethane/petroleum spirit 3:1): Rf=0,195. NMR 1H (300 MHz, CDCl3): 4.401 (s, 5H, H1 ferrocene), 4.355 (s, 2H, H2 or H3 ferrocene), 4.459 (s, 2H, H2 or H3 ferrocene), 7.70 (m, 2H, H6 and H7 aromatic), 6.906 (d, 1H, H4 or H5 vinyl, J=13.8 MHz), 8.764 (d, 1H, H4 or H5 vinyl, J=15.0 MHz). MS (APCI) m/z 440.15 [M]+°. Yield=84%.
**9e** : Green solid. TLC (dichloromethane/petroleum spirit 3:1): Rf=0,256. MS (APCI) m/z 538.09 [M]+°. Yield=67%.
**9f** : Green solid. TLC (dichloromethane/petroleum spirit 3:1): Rf=0,273. NMR 1H (300 MHz, CDCl3): 4.395 (s, 5H, H1 ferrocene), 4.892 (s, 2H, H2 or H3 ferrocene), 5.147 (s, 2H, H2 or H3 ferrocene), 6.856 (d, 1H, H4 or H5 vinyl, J=11,1 MHz), 7.0 (m, 2H, H6 and H7 aromatic), 8.759 (d, 1H, H4 or H5 vinyl, J=11,5 MHz). MS (APCI) m/z 416.02 [M]+°. Yield=54%.
**9g :** Green solid. TLC (dichloromethane/petroleum spirit 3:1): Rf=0,128. NMR 1H (300 MHz, CDCl3): 3.786 (s, 3H, H9 methoxy), 4.270 (s, 5H, H1 ferrocene), 4.766 (s, 2H, H2 or H3 ferrocene), 4.892 (s, 2H, H2 or H3 ferrocene), 6.877 (d, 1H, H4 or H5 vinyl, J=14.1 MHz), 6.941 (s, 1H, H8 aromatic), 6.984 (d, 1H, H6 aromatic, J=7.6 MHz), 7.289 (d, 1H, H7 aromatic, J=8.1 MHz), 8.477 (d, 1H, H4 or H5 vinyl, J=12.3 MHz) NMR 13C (75 MHz, CDCl3): 56.2 (Cquat methoxy), 71.6 (C1 ferrocene), 78.8 (Cquat ferrocene), 108.4 (C aromatic), 111.2 (C aromatic), 116.0 (Cquat aromatic), 122.4 (C aromatic), 130.8 (C vinyl), 152.8 (Cquat aromatic), 159.1 (Cquat aromatic), 159.4 (C vinyl), 182.4 (Cquat ketone). MS (APCI) m/z 410.08 [M]+°.Yield=23%.
**9h** : Green solid. TLC (dichloromethane/petroleum spirit 3:1): Rf=0,156. NMR 1H (300 MHz, CDCl3): 3.743 (s, 6H, H8 and H9 methoxy), 3.885 (s, 5H, H1 ferrocene), 4.529 (s, 2H, H2 or H3 ferrocene), 4.585 (s, 2H, H2 or H3 ferrocene), 5.801 (d, 1H, H7 aromatic, J=9.3 MHz), 6.003 (d, 1H, H6 aromatic, J=13,1 MHz), 7.393 (d, 1H, H4 or H5 vinyl, J=14.7 MHz), 8.220 (d, 1H, H4 or H5 vinyl, J=15.0 MHz) NMR 13C (75 MHz, CDCl3): 56.2 (Cquat methoxy), 70.7 (C1 ferrocene), 79.0 (Cquat ferrocene), 93.0 (C aromatic), 93.3 (C aromatic), 94.8 (Cquat aromatic), 95.4 (C vinyl), 117.9 (Cquat aromatic), 155.6 (Cquat aromatic), 196.0( Cquat ketone). MS (APCI) m/z 410.07 [M]+°.Yield=17%.
**9i** : Green solid. TLC (dichloromethane/petroleum spirit 3:1): Rf=0,205. NMR 1H (300 MHz, CDCl3): 3.839 (d, 3H, H9 methoxy, J=20,7 MHz), 4.212 (s, 5H, H1 ferrocene), 4.545 (s, 2H, H2 or H3 ferrocene), 4.732 (s, 2H, H2 or H3 ferrocene), 6.902 (d, 1H, H4 or H5 vinyl, J=14,1 MHz), 6.452 (s, 1H, H6 aromatic), 6.510 (d, 1H, H8 aromatic, J=9.6 MHz), 7.565 (d, 1H, H7 aromatic, J=8,5 MHz), 8.374 (d, 1H, H4 or H5 vinyl, J=14,1 MHz). NMR 13C (75 MHz, CDCl3): 55.6 (Cquat methoxy), 70.9 (C1 ferrocene), 77.2 (Cquat ferrocene), 101.9 (C aromatic), 111.5 (Cquat aromatic), 112.2 (C aromatic), 130.6 (C vinyl), 132.3 (C aromatic), 156.6 (C vinyl). MS (APCI) m/z 414.04 [M]+°. Yield=16%.
**9j** : Green solid. TLC (dichloromethane/petroleum spirit 3:1): Rf=0,188. NMR 1H (300 MHz, CDCl3): 3.785 (s, 3H, H9 methoxy), 4.087 (s, 5H, H1 ferrocene), 4.536 (s, 2H, H2 or H3 ferrocene), 4.651 (s, 2H, H2 or H3 ferrocene), 6.209 (d, 1H, H8 aromatic, J=7.9 MHz), 6.465 (d, 1H, H6 aromatic, J=8,1 MHz), 7,360 (m, 1H, H7 aromatic), 7.400 (d, 1H, H4 or H5 vinyl, J=14.7 MHz), 8.333 (d, 1H, H4 or H5 vinyl, J=14.4 MHz) NMR 13C (75 MHz, CDCl3): 53.4 (Cquat methoxy), 56.3 - 71.0 (C1 ferrocene), 79.0 (Cquat ferrocene), 102.0 (C aromatic), 113.3 (C vinyl), 117.8 (C aromatic), 140.8 (C aromatic), 158.8 (C aromatic), 158.6 (C vinyl), 161.7 (Cquat aromatic), 164.8 (Cquat aromatic), 182.5 (Cquat ketone). MS (APCI) m/z 410.07 [M]+°. Yield=57%.

### Example 7: antiproliferative effects in vitro

Murine B16 melanoma cells were grown in Dulbecco's modified essential medium (DMEM) containing 2 mM L-glutamine, 10% fetal bovine serum, 100 U/ml penicillin and 100 µg/ml streptomycin (37°C, 5% CO₂). Stock solutions of the compounds were prepared in DMSO and further diluted in DMEM at the indicated concentrations with a final DMSO concentration of not exceeding 1%. Exponentially growing cells were plated onto 96-well plates at a density of 5000 cells per well in 200 µl DMEM, and 24 h later the compounds were added for another 48 h. Control wells were exposed to 1% DMSO. Viability was assessed using the MTT (1-(4,5-dimethylthiazol-2-yl)-3,5-diphenyltetrazolium) test and absorbance was read at 562 nm in a microplate reader (BioKinetics Reader, EL340) (Carmichael et al. 1987). Results are presented as the inhibitory concentrations for 50% of cells (IC50) (mean ± SD of 3 determinations) for a 48 h exposure time.

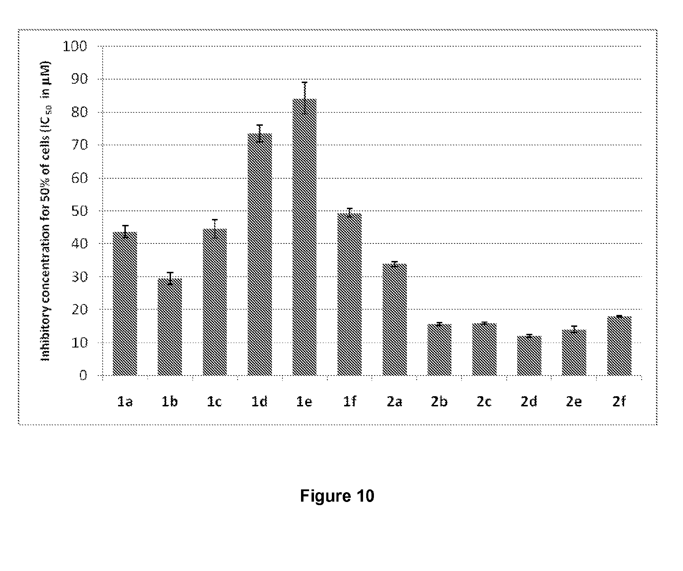

Compounds 1a-f and 2a-f were evaluated for their cytotoxicity against the murine B16 melanoma cancer cell line. The chalcone-derived compounds presented inhibitory concentrations for 50% of cells (IC50) in the range of 30 to 84 µM (Table 1 below). Figure 10 illustrates the ferrocenyl flavonoids cytotoxicity on murine B16 melanoma cells.

**Table 1.**

| Compound | IC50 |
|---|---|
| 1a | 43.7 ± 1.8 |
| 1b | 29.5 ± 1.8 |
| 1c | 44.6 ± 2.8 |
| 1d | 73.6 ± 2.6 |
| 1e | 84.2 ± 4.8 |
| 1f | 49.4 ± 1.3 |
| 2a | 33.9 ± 0.8 |
| 2b | 15.6 ± 0.3 |
| 2c | 15.9 ± 0.3 |
| 2d | 12.1 ± 0.4 |
| 2e | 13.7 ± 1.0 |
| 2f | 18.0 ± 0.2 |

In this series, compounds 1d, 1e, and 1f, bearing 2 halogens in the meta position of cycle A, presented a decrease in cytotoxic activity. For the flavone-derived ferrocenyl compounds (2a-f), the presence of halogens on the A cycle markedly increased the cytotoxic activity, by decreasing the IC50 of the reference compound 2a (lacking halogens) by a factor of 2.

It is of interest that most ferrocenyl flavonoids bearing halogens on cycle A presented IC50 values in the low micromolar range (12 - 18 µM), which are among the lowest IC50 reported amongst the flavone family (Touil et al. 2009).

## Claims

1. Compound of formula (I): 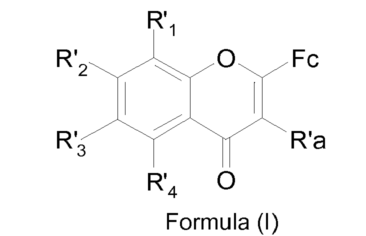 wherein
Fc is ferrocenyl,
R'a is hydrogen, a halogen, a C1-C6 alkyl, a C2-C6 alkenyl or a -CO-R'₅ group,
R'₁ is hydrogen, a halogen, hydroxy, nitro, a C1-C6 alkyl, a C2-C6 alkenyl or R'₁ together with R'₂ is a C6-C14 aryl,
R'₂ is hydrogen, a halogen, hydroxy, amino (-NH2), a C1-C6 alkyl, a C2-C6 alkenyl, -OR'₆, -NH-CO-R'₆, -O-CO-R'₆ or R'₂ together with R'₁ is a C6-C14 aryl,
R'₃ is hydrogen, a halogen, hydroxy, a C1-C6 alkyl, a C2-C6 alkenyl, -COOH, - OR'₇, -NH-CO-R'₇, -CO-R'₇ or R'₃ together with R'₄ is a C6-C14 aryl,
R'₄ is hydrogen, hydroxy, a C1-C6 alkyl, a C2-C6 alkenyl, -OR'₈ or R'₄ together with R'₃ is a C6-C14 aryl,
R'₅, R'₆, R'₇ and R'₈ are the same or different and are independently selected from a C1-C6 alkyl, a C2-C6 alkenyl or a C6-C14 aryl.

2. Compound of formula (II): 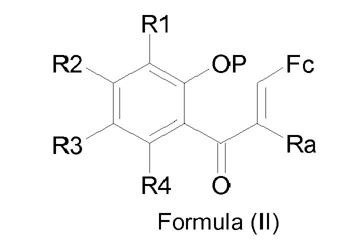 wherein
Fc is ferrocenyl,
P is hydrogen, an alcohol protecting group or -BF₂,
Ra is hydrogen, a halogen, a C1-C6 alkyl, a C2-C6 alkenyl or a -CO-R5 group,
R1 is hydrogen, a halogen, hydroxy, nitro, a C1-C6 alkyl, a C2-C6 alkenyl or R1 together with R2 is a C6-C14 aryl,
R2 is hydrogen, a halogen, hydroxy, amino (-NH2), a C1-C6 alkyl, a C2-C6 alkenyl, -OR6, -NH-CO-R6, -O-CO-R6 or R2 together with R1 is a C6-C14 aryl,
R3 is hydrogen, a halogen, hydroxy, a C1-C6 alkyl, a C2-C6 alkenyl, -COOH, - OR7, -NH-CO-R7, -CO-R7 or R3 together with R4 is a C6-C14 aryl,
R4 is hydrogen, hydroxy, a C1-C6 alkyl, a C2-C6 alkenyl, -OR8 or R4 together with R3 is a C6-C14 aryl,
R5, R6, R7 and R8 are the same or different and are independently selected from a C1-C6 alkyl, a C2-C6 alkenyl or a C6-C14 aryl.
with the proviso that the following compounds are excluded:
- 3-ferrocenyl-1-(2-hydroxyphenyl)-prop-2-en-1-one,
- 3-ferrocenyl-1-(2,4-dihydroxyphenyl)-prop-2-en-1-one,
- 3-ferrocenyl-1-(2,4-dimethoxyphenyl)-prop-2-en-1-one,
- 3-ferrocenyl-1-(2,3,4-trimethoxyphenyl)-prop-2-en-1-one.

3. Compound according to claim 2, wherein
- P is -BF₂,
- Ra is hydrogen,
- R1 is hydrogen or a halogen,
- R2 is -OR6,
- R3 is hydrogen, a halogen or-OR7,
- R4 is -OR8,
- R6, R7 and R8 are the same or different and are independently a C1-C6 alkyl.

4. Method of preparation of compounds according to claim 1, by oxidation of a compound of formula (III): 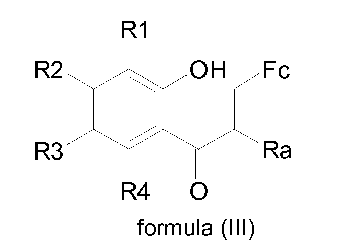 wherein
Fc is ferrocenyl,
Ra is hydrogen, a halogen, a C1-C6 alkyl, a C2-C6 alkenyl or a -CO-R5 group,
R1 is hydrogen, a halogen, hydroxy, nitro, a C1-C6 alkyl, a C2-C6 alkenyl or R1 together with R2 is a C6-C14 aryl,
R2 is hydrogen, a halogen, hydroxy, amino (-NH2), a C1-C6 alkyl, a C2-C6 alkenyl, -OR6, -NH-CO-R6, -O-CO-R6 or R2 together with R1 is a C6-C14 aryl,
R3 is hydrogen, a halogen, hydroxy, a C1-C6 alkyl, a C2-C6 alkenyl, -COOH, - OR7, -NH-CO-R7, -CO-R7 or R3 together with R4 is a C6-C14 aryl,
R4 is hydrogen, hydroxy, a C1-C6 alkyl, a C2-C6 alkenyl, -OR8 or R4 together with R3 is a C6-C14 aryl,
R5, R6, R7 and R8 are the same or different and are independently selected from a C1-C6 alkyl, a C2-C6 alkenyl or a C6-C14 aryl. in the presence of strong base and an oxidizing agent.

5. Method according to claim 4, wherein the strong base is sodium hydride and the oxidizing agent is silver triflate.

6. Method according to claim 5 or 6, wherein the oxidation is conducted in an organic solvent chosen among THF and ethanol.

7. Method of preparation of a compound according to claim 2 wherein P is -BF₂, by reacting a compound of formula (III): 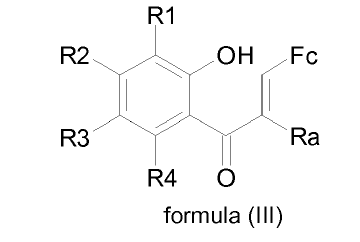 wherein
Fc is ferrocenyl,
Ra is hydrogen, a halogen, a C1-C6 alkyl, a C2-C6 alkenyl or a -CO-R5 group,
R1 is hydrogen, a halogen, hydroxy, nitro, a C1-C6 alkyl, a C2-C6 alkenyl or R1 together with R2 is a C6-C14 aryl,
R2 is hydrogen, a halogen, hydroxy, amino (-NH2), a C1-C6 alkyl, a C2-C6 alkenyl, -OR6, -NH-CO-R6, -O-CO-R6 or R2 together with R1 is a C6-C14 aryl,
R3 is hydrogen, a halogen, hydroxy, a C1-C6 alkyl, a C2-C6 alkenyl, -COOH, - OR7, -NH-CO-R7, -CO-R7 or R3 together with R4 is a C6-C14 aryl,
R4 is hydrogen, hydroxy, a C1-C6 alkyl, a C2-C6 alkenyl, -OR8 or R4 together with R3 is a C6-C14 aryl,
R5, R6, R7 and R8 are the same or different and are independently selected from a C1-C6 alkyl, a C2-C6 alkenyl or a C6-C14 aryl,
with a strong base, and adding to the reaction mixture boron trifluoride diethyl etherate and a strong acid.

8. Method according to claim 7, wherein the strong base is sodium hydride and the strong acid, hydrochloric acid.

9. Compounds according to any of claims 1 to 3, or a pharmaceutically acceptable salt thereof, for use as a medicament.

10. Use of the compounds according to any of claims 1 to 3, or a pharmaceutically acceptable salt thereof, for the manufacture of a medicament for the treatment of cancer.
